# EUROPEAN PATENT APPLICATION

(11) **EP 1 870 107 A1**
(43) Date of publication of application: **26.12.2007**
(21) Application number: 06291034.4
(22) Date of filing: 23.06.2006
(51) Int. Cl.: A61K 38/21, A61P 25/28

(54) **Use of inducer of promyelocytic leukemia protein (PML) for treating polyglutamine expansion neurodegenerative diseases**

(71) Applicant: INSERM (Institut National de la Santé et de la Recherche Médicale), 75654 Paris Cedex 13 (FR)
(72) Inventor: The designation of the inventor has not yet been filed
(74) Representative: Chajmowicz, Marion

(57) **Abstract**

The present invention relates to use of an inducer of Promyelocytic Leukemia protein (PML) expression, and especially PML IV for the manufacture of a medicament intended for the treatment of polyglutamine expansion neurodegenerative diseases. More specifically, the present invention relates to the use of an interferon polypeptide for the manufacture of a medicament intended for the treatment of polyglutamine expansion neurodegenerative diseases.

## Description

The present invention relates to the use of an inducer of Promyelocytic Leukemia protein (PML) expression for the manufacture of a medicament intended for the treatment of a polyglutamine expansion neurodegenerative disease. More specifically, the present invention relates to the use of an interferon polypeptide for the manufacture of a medicament intended for the treatment of polyglutamine expansion neurodegenerative diseases.

Polyglutamine expansion neurodegenerative diseases, including spinocerebellar ataxia 1, 2, 3, 6, 7, and 17, Huntington's disease, spinal and bulbar muscular atrophy (SBMA), and dentatorubral pallidoluysian atrophy (DRPLA) are hereditary neurodegenerative disorders caused by CAG repeat expansions encoding polyglutamine stretches in the corresponding proteins (Fujigasaki et al 2001, Nakamura et al 2001 and Zoghbi and Orr 2000). These CAG repeat expansions result in the synthesis and accumulation of ubiquitouslly expressed polyglutamine-containing proteins, some of still unknown function (e.g. Huntingtin in Huntington's disease and ataxins in spinocerebellar ataxias). The accumulation of polyglutamine-containing proteins in various brain regions is responsible for neurologic problems. Proteins with expanded polyglutamine domains aggregate and indeed aggregation is a pathologic hallmark of the polyglutamine repeat diseases (Hackam et al. 1998; Perez et al. 1998)).

All diseases in the CAG repeat family show genetic anticipation, meaning that the disease usually appears at an earlier age and increases in severity with each generation. Genetic anticipation is linked to increasing numbers of CAG repeats, which result from expansion of the unstable CAG sequence when reproductive cells divide to form eggs and sperm. In general, neurodegenerative disorders are progressive (i.e., their symptoms are not apparent until months or more commonly years after the disease has begun), and caused by an initial reduction of neuronal function, followed by a complete dysfunction upon neuronal death.

Huntington's Disease (HD) is a devastating, degenerative brain disorder for which there is, at present, no effective treatment or cure. Early symptoms of Huntington's Disease may affect cognitive ability or mobility and include depression, mood swings, forgetfulness, clumsiness, involuntary twitching and lack of coordination. As the disease progresses, concentration and short-term memory diminish and involuntary movements of the head, trunk and limbs increase. Walking, speaking and swallowing abilities deteriorate. Eventually, the person with HD becomes totally dependent upon others for his or her care. Death follows from complications such as choking, infection or heart failure. HD typically begins in midlife, between the ages of 30 and 45, though onset may occur as early as the age of 2 in its juvenile, more severe form. Children who develop the juvenile form of the disease rarely live to adulthood. HD affects males and females equally and crosses all ethnic and racial boundaries. Each child of a person with HD has a 50/50 chance of inheriting the fatal gene. HD is an autosomal dominant condition and thus everyone who carries the mutant allele of the gene will develop the disease.

The Huntington's Disease (HD) gene was cloned in 1993 (The Huntington's Disease Collaborative Research Group. Cell. 1993) and found to contain a CAG repeat within its 5'-end coding sequence. This CAG repeat is expanded in individuals with HD (who may or may not be symptomatic, depending upon their age). However, the presence of a CAG repeat expansion is found in virtually all symptomatic HD individuals. Individuals affected with HD typically have at least one HD allele with 36 or more CAG repeat. The polyglutamine expansion results in the formation of insoluble, high molecular weight protein aggregates similar to those seen in Alzheimer's disease (Scherzinger et al, 1997).

Spinobulbar muscular atrophy (SBMA, also named Kennedy disease) is caused by a specific mutation (an expansion of the normally polymorphic CAG trinucleotide repeat) in the first exon of the androgen receptor gene (which encodes polyglutamine tracts) which is located on the X-chromosone. Similar to Huntington's disease, in this disease CAG is also abnormally repeated. The CAG repeat range in the general population is approximately 12 to 32 repeats. In patients with Kennedy disease, the repeats may vary from 40 to 55 repeats. Symptoms appear when the repeats exceed about 40. A larger number of repeats has been suggested to cause symptoms to begin earlier in life and progress more rapidly.

Spinocerebellar ataxias are a group of autosomal dominantly inherited ataxias with heterogeneous presentation. Characteristic CAG repeat expansions in the coding sequences at several loci have been detected for certain of these disorders. For example, Spinocerebellar ataxia 7 (SCA7) is a progressive autosomal dominant neurodegenerative disorder characterized by cerebellar ataxia and visual impairment (David et al., 1997) due to moderate to severe neuronal loss in the cerebellum and associated structures and degeneration of cone and rod photoreceptors. The function of the protein encoded by the SCA7 gene was elucidated, in 2004. The SCA7 gene product, ataxin-7 (ATXN7), is a component of the TBP-free TAF-containing complex (TFTC) and the SPT3/TAF9/GCN5 acetyltransferase complex (STAGA), which are implicated in several steps of transcriptional regulation, such as histone acetylation and recruitment of the preinitiation complex to promoters (Helmlinger et al., 2004b; Scheel et al., 2003).

Current treatment of the polyglutamine expansion neurodegenerative diseases includes number of medications to help control some symptoms like emotional and movement problems associated with polyglutamine disorders. However, there is currently no efficient treatment to stop or reverse the course of the polyglutamine expansion neurodegenerative diseases. Thus, new methods for the treatment of neurodegenerative polyglutamine diseases, including but not limited to Huntington's disease, spinocerebellar ataxias, and spinobulbar muscular atrophy (Kennedy's Disease) that are effective and convenient are really needed.

An understanding of the mechanisms whereby mutant proteins accumulate, aggregate or are eliminated in the nucleus would therefore help in the development of therapeutic strategies for these diseases.

Regarding the Spinocerebellar ataxias, previous studies showed that PML (Promyelocytic Leukemia protein) nuclear bodies (NBs) colocalized with polyQ-containing proteins in nuclear matrix preparations of cells expressing ATXN7 or ataxin-1 (Kaytor et al., 1999; Skinner et al., 1997). Furthermore, the normal nuclear distribution of PML was altered by the expression of mutant ataxin-1 and ataxin-3 (Chai et al., 1999; Skinner et al., 1997). In the brains of patients with SCA7 or other polyQ disorders, PML bodies often colocalized with neuronal intranuclear inclusions (Takahashi et al., 2003). Interestingly, colocalization of PML bodies occurred more frequently in small than in large Nls (Takahashi et al., 2002), suggesting that PML bodies are associated with early steps of polyQ protein aggregation.

PML bodies are multiprotein complexes distributed in a speckled pattern throughout the nucleus (Borden, 2002; Jensen et al., 2001; Negorev and Maul, 2001), where they have been suggested to play a role in many cellular processes, such as transcriptional regulation, growth control and apoptosis (Seeler and Dejean, 1999; Zhong et al., 2000). The heterogeneous composition and morphology of PML bodies have led to the suggestion that the different PML isoforms have distinct cellular functions (Beech et al., 2005). Because of the variety of partner proteins found in PML bodies, it has also been proposed that PML bodies might be storage compartments for nuclear proteins (Negorev and Maul, 2001). However, a subset of PML bodies, called clastosomes, contain components of the UPS and were suggested to be sites of protein degradation in the nucleus (Lafarga et al., 2002). Clastosomes appear to be transient structures that assemble when proteolysis is highly active in the nucleus, but disappear when the proteasome is inhibited (Lafarga et al., 2002). Interestingly, Muratani and co-workers showed that some PML bodies move about rapidly in the nucleus in an energy-dependent fashion (Muratani et al., 2002), and suggested that these dynamic PML bodies may be nuclear sensors of foreign or misfolded proteins, including those of viral origin, and would thus act like a subnuclear immune system. This is consistent with the observation that interferon (INF) induces PML expression and stimulates the formation of PML bodies (Regad and Chelbi-Alix, 2001). However despite that PML nuclear bodies were shown to be present in polyQ aggregates, their relation to pathogenesis remains unclear to the skilled man in the art.

Now, the present invention provides a new method for the treatment of the polyglutamine expansion neurodegenerative diseases. The inventors indeed demonstrated that expression of PML isoform IV leads to the formation of distinct nuclear bodies enriched in components of the ubiquitin-proteasome system. These bodies recruit soluble mutant ataxin-7 and promote its degradation by proteasome-dependent proteolysis, thus preventing the aggregate formation. Inversely, disruption of the endogenous nuclear bodies with cadmium increases the nuclear accumulation and aggregation of mutant ataxin-7, demonstrating their role in ataxin-7 turn-over. Interestingly, interferon-beta treatment, which induces the expression of endogenous PML IV, prevents the accumulation of transiently expressed mutant ataxin-7, without affecting the levels of the endogenous wild type protein.

Therefore an object of the invention thus relates to the use of an inducer of PML expression for the manufacture of a medicament intended for treating a polyglutamine expansion neurodegenerative disease.

A second object of the invention relates to a method of treatment of polyglutamine expansion neurodegenerative diseases in a mammal comprising the administration of a therapeutically effective amount of at least one inducer of PML expression to a subject in need thereof.

### Definitions

A "coding sequence" or a sequence "encoding" an expression product, such as an RNA, polypeptide, protein, or enzyme, is a nucleotide sequence that, when expressed, results in the production of that RNA, polypeptide, protein, or enzyme, i.e., the nucleotide sequence encodes an amino acid sequence for that polypeptide, protein or enzyme. A coding sequence for a protein may include a start codon (usually ATG) and a stop codon.

As used herein, references to specific proteins (e.g., PML and interferon) can include a polypeptide having a native amino acid sequence, as well as variants and modified forms regardless of their origin or mode of preparation. A protein that has a native amino acid sequence is a protein having the same amino acid sequence as obtained from nature (e.g., a naturally occurring PML or interferon). Such native sequence proteins can be isolated from nature or can be prepared using standard recombinant and/or synthetic methods. Native sequence proteins specifically encompass naturally occurring truncated or soluble forms, naturally occurring variant forms (e.g., alternatively spliced forms), naturally occurring allelic variants and forms including postranslational modifications. A native sequence protein includes proteins following post-translational modifications such as glycosylation, or phosphorylation, or other modifications of some amino acid residues.

Variants refer to proteins that are functional equivalents to a native sequence protein that have similar amino acid sequences and retain, to some extent, one or more activities of the native protein. Variants also include fragments that retain activity. Variants also include proteins that are substantially identical (e.g., that have 80, 85, 90, 95, 97, 98, 99%, sequence identity) to a native sequence. Such variants include proteins having amino acid alterations such as deletions, insertions and/or substitutions. A "deletion" refers to the absence of one or more amino acid residues in the related protein. The term "insertion" refers to the addition of one or more amino acids in the related protein. A "substitution" refers to the replacement of one or more amino acid residues by another amino acid residue in the polypeptide. Typically, such alterations are conservative in nature such that the activity of the variant protein is substantially similar to a native sequence protein (see, e.g., Creighton (1984) Proteins, W.H. Freeman and Company). In the case of substitutions, the amino acid replacing another amino acid usually has similar structural and/or chemical properties. Insertions and deletions are typically in the range of 1 to 5 amino acids, although depending upon the location of the insertion, more amino acids can be inserted or removed. The variations can be made using methods known in the art such as site-directed mutagenesis (Carter, et al. (1986) Nucl. Acids Res. 13:4331; Zoller et al. (1987) Nucl. Acids Res. 10:6487), cassette mutagenesis (Wells et al. (1985) Gene 34:315), restriction selection mutagenesis (Wells, et al. (1986) Philos. Trans. R. Soc. London SerA 317:415), and PCR mutagenesis (Sambrook et al., Molecular Cloning: A Laboratory Manual, 3rd edition, Cold Spring Harbor Press, N.Y., (2001)).

Two amino acid sequences are "substantially homologous" or "substantially similar" when greater than 80 %, preferably greater than 85 %, preferably greater than 90 % of the amino acids are identical, or greater than about 90 %, preferably grater than 95 %, are similar (functionally identical). Preferably, the similar or homologous sequences are identified by alignment using, for example, the GCG (Genetics Computer Group, Program Manual for the GCG Package, Version 7, Madison, Wisconsin) pileup program, or any of sequence comparison algorithms such as BLAST, FASTA, etc.

The term "expression" when used in the context of expression of a gene or nucleic acid refers to the conversion of the information, contained in a gene, into a gene product. A gene product can be the direct transcriptional product of a gene (e.g., mRNA, tRNA, rRNA, antisense RNA, ribozyme, structural RNA or any other type of RNA) or a protein produced by translation of a mRNA. Gene products also include messenger RNAs which are modified, by processes such as capping, polyadenylation, methylation, and editing, and proteins (e.g., PML) modified by, for example, methylation, acetylation, phosphorylation, ubiquitination, SUMOylation, ADP-ribosylation, myristilation, and glycosylation.

The terms "up-regulated and "activation" when used in reference to the expression of a nucleic acid such as a gene (e.g., PML) refers to any process which results in an increase in production of a gene product or activity of the gene product. A gene product can be either RNA (including, but not limited to, mRNA, rRNA, tRNA, and structural RNA) or protein (e.g., PML) Accordingly, gene up-regulation or activation includes those processes that increase transcription of a gene and/or translation of a mRNA. The level of gene expression, including the level of gene activation or up-regulation, can be quantitated utilizing a number of established techniques including, but not limited to, Northern-Blots, RNase protection assays (RPA), nucleic acid probe arrays, quantitative PCR (e.g., the so-called TaqMan assays), dot blot assays and in-situ hybridization. In general, gene up-regulation or activation comprises any detectable increase in the production of a gene product, preferably an increase in production of a gene product by at least 30, 40, 50 or 100%.

An "inducer of expression" denotes a compound, natural or not, which has the capability to up regulate or activate the expression of a gene and consequently the expression of the corresponding protein. Therefore an "inducer of PML expression" denotes a compound, natural or not, which has the capability to up regulate or activate the expression of a PML gene and consequently expression of a PML polypeptide. Such inducers of PML expression include interferons, as well as p53 activators, i.e. compounds that activate p53 or induce the expression of p53 (de Stanchina et al, 2004; Fogal et al 2000; .Môller et al, 2003; Coutts et al, 2005; Everett et al, 2006).

A " Promyelocytic Leukemia protein" or "PML", as used herein, is intended to include any molecule defined as such in the literature, comprising for example any types of PML and in particular, PML IV. The PML gene consist of nine exons and several alternative spliced PML transcripts have been described (de Thé et al., 1991; Fagioli et al., 1992; Goddard et al., 1991; Kakizuka et al., 1991; Kastner et al., 1992). PML belongs to a family of proteins defined by the presence of the RBCC motif, a C3HC4 (RING finger) zinc binding motif, one or two other cysteine-rich motifs, the B boxes and a coiled-coil region. PML is localisedin the nuclear diffuse fraction of the nucleoplasm and in a discrete sub-nuclear compartment, the nuclear bodies (NBs) (Daniel et al., 1993; Dyck et al., 1994; Koken et al., 1994; Weis et al., 1994). The PML isoforms can be divided into seven groups, which we are designating as PML I-VII, on the basis of sequence differences, due to alternative splicing, at the C-terminus. It has been shown that PML III and PML IV exist without exon 5 (de Thé et al., 1991; Fagioli et al., 1992) and that PML V can exist without exons 5 and 6 or 4, 5 and 6 (Fagioli et al., 1992), although the sequences for the latter two isoforms are not available. Table I provides for the description of the PML isoforms with their corresponding accession numbers.

**Table I : Nomenclature for PML isoforms and alternative spliced variants**

| *Isoforms* | | *References* | *Accession* |
|---|---|---|---|
| PML I | 882aa | PML4 (Fagioli *et al.,* 1992) | |
| | 882aa | PML- I (Goddard er *al*., 1991) | M79462 |
| | 882aa | TRIM19 alpha (Reymond *et al.,* 2001) | AF230401 |
| PML II. | 829aa | PML2 (Fagioli *et al*., 1992) | |
| | 824aa | PML-3 (Goddard *et al.,* 1991) | M79464 |
| | 824aa | TRIM19 gamma (Reymond *et al*., 2001) | AF230403 |
| | 854aa | TRIM19 delta¹ (Reymond *et al*., 2001) | AF230404 |
| | 829aa | TRIM19 kappa (Reymond *et al.,* 2001) | AF230410 |
| PML III | 641 aa | PML-L(deThe *et al*., 1991) | S50913 |
| PML IIIa | 593aa | PML-S (de The *et al*., 1991) | |
| PML IV | 633aa | PMI.3 (Fagioli *et al.,* 1992) | |
| | 633aa | Myl (Kastner *et al.,* 1992) | X63131 |
| | 633aa | TRIM19 zeta (Reymond *et al.,* 2001) | AF230406 |
| PML IVa | 585aa | TRIM19 lambda (Reymond *et al., 2001)* | AF230411 |
| PML V | 611aa | PML1 (Fagioli *et al.,* 1992) | |
| | 611aa | PML-2 (Goddard *et al..* 1991) | M79463 |
| | 611aa | TRIM19 beta (Reymond *et al.,* 2001) | AF230402 |
| PML VI | 560aa | PML-1 (Kakizuka *et al.,* 1991) | M73778 |
| | 560aa | PML-3B (Goddard *et al.,* 1991) | M80185 |
| | 560aa | TRIM19 epsilon (Reymond *et al*., 2001) | AF230405 |
| PML VIb | 423aa | TRIM19 iota² (Reymond *et al*., 2001) | AF230409 |
| PML VIb | 423aa | TRIM19 eta² (Reymond *et al*., 2001) | AF230407 |
| PML VIIb | 435aa | TRIM19 theta (Reymond *et al*., 2001) | AF230408 |

An "interferon" or "IFN", as used herein, is intended to include any molecule defined as such in the literature, comprising for example any types of IFNs (type I and type II) and in particular, IFN-alpha, IFN-beta, INF-omega and IFN-gamma. IFNs were actually discovered in 1957 (Isaacs & Lindenmann, 1957). The term, interferon, originally described the biological activity of a soluble substance that "interfered" with viral replication in cultured cells. Other biological roles of interferons emerged later, including antiproliferative and immunomodulatory effects. IFNs are naturally occurring proteins produced by cells (leukocytes, fibroblasts, T lymphocytes, and natural killer (NK) cells, in response to antigenic stimulation with viral RNA, bacterial products, or tumor proteins. IFNs are considered part of the innate immune system. However, they can influence adaptive responses of the immune system, including B and T cell differentiation. Early work identified 3 different classes of IFNs based on the cells of origin. Leukocytes exposed to viruses secreted IFN-alpha, stimulated fibroblasts produced IFN-beta, and activated lymphocytes T mainly secreted IFN-gamma. Based on similar acid stability, homology, and function, the leukocyte and fibroblast IFNs were collectively called type I IFNs. Distinct from type I IFNs in terms of function and acid lability was IFN-gamma, also known as type II IFN. Later studies identified other type I IFNs, such as IFN-ω, IFN-κ, and IFN-T (Charlier et al., 1993, Rueda et al., 1993 and LaFleur et al., 2001). Some classes of IFNs contain several related genes. The IFN-α class makes up at least 18 nonallelic genes located on the short arm of chromosome 9. The w IFN class contains 6 nonallelic genes also located on chromosome 9. IFN-β and IFN-γ exist in only 1 allelic form and are located on chromosomes 9 and 12. The term interferon, as used herein, is also intended to encompass salts, functional derivatives, variants, muteins, fused proteins, analogs and active fragments thereof.The polypeptide sequences for human interferon-alpha are deposited in database under accession numbers : AAA 52716 , AAA 52724, and AAA 52713. The polypeptide sequences for human interferon-beta are deposited in database under accession numbers AAC41702, NP_002167, AAH 96152, AAH 96153, AAH 96150, AAH 96151, AAH 69314, and AAH 36040. The polypeptide sequences for human interferon-gamma are deposited in database under accession numbers AAB 59534, AAM 28885, CAA 44325, AAK 95388, CAA 00226, AAP 20100, AAP 20098, AAK 53058, and NP-000610.

By "purified" and "isolated" it is meant, when referring to a polypeptide (i.e. interferon) or a nucleotide sequence, that the indicated molecule is present in the substantial absence of other biological macromolecules of the same type. The term "purified" as used herein preferably means at least 75% by weight, more preferably at least 85% by weight, still preferably at least 95% by weight, and most preferably at least 98% by weight, of biological macromolecules of the same type are present. An "isolated" nucleic acid molecule which encodes a particular polypeptide refers to a nucleic acid molecule which is substantially free of other nucleic acid molecules that do not encode the subject polypeptide; however, the molecule may include some additional bases or moieties which do not deleteriously affect the basic characteristics of the composition.

A "polyglutamine expansion neurodegenerative disease" or "'polyQ expansion neurodegenerative disease" is a neurodegenerative disease or disorder which is caused by CAG repeat expansions in the gene, encoding polyglutamine (polyQ) stretches in the corresponding protein.

As used herein, the term "subject" denotes a mammal, such as a rodent, a feline, a canine, and a primate. Preferably a subject according to the invention is a human.

### Therapeutic methods and uses

The present invention provides for methods and compositions (such as pharmaceutical compositions) for treating polyglutamine expansion neurodegenerative diseases. The polyglutamine expansion neurodegenerative diseases include spinocerebellar ataxia 1, 2, 3, 6, 7, and 17, Huntington's disease, spinal and bulbar muscular atrophy (SBMA or Kennedy disease), and dentatorubral pallidoluysian atrophy (DRPLA).

An object of the invention is the use of an inducer of PML expression for the manufacture of a medicament intended for treating a polyglutamine expansion neurodegenerative disease. In a preferred embodiment, said inducer of PML expression is an inducer of PML IV expression.

In a particular embodiment, the inducer of PML expression is chosen among interferon polypeptides, including all types of interferons such as alpha, beta, omega and gamma interferon polypeptides. In a preferred embodiment the inducer of PML expression includes interferon-beta polypeptides, and specifically interferon beta-1a or interferon beta-1b polypeptides. In a most preferred embodiment, the inducer of PML expression is an interferon beta-1a polypeptide.

The term "interferon-beta-1a" or "IFN-β-1a" as used herein refers to a glycosylated form of IFN-beta polypeptide at residue 80 (Asn 80). The term "interferon-beta-1a" is also meant to encompass mutants thereof, provided that such mutants are also glycosylated at residue 80 (Asn 80). Recombinant DNA methods for producing proteins, including interferons are known (see for example, U.S. Patents 4,399,216, 5,149,636, 5,179,017,and 4,470,461). Preferred interferon-beta-1a polypeptides that may be used in the present methods are derived from the wild-type interferon beta gene sequences of various vertebrates, preferably mammals, and more preferably human, and are obtained using methods that are well-known to those having ordinary skill in the art such as the methods described in the following U.S. Patents: U.S Patent 5,641,656, U.S. Patent 5,605,688; U.S. Patent 5,231,176; U.S. Patent 5,071,761; U.S. Patent 4,970,161; U.S. Patent 4,738,931; U.S. Patent 4,695,543 and U.S. Patent 4,456,748. Mutants of interferon-beta-1a may be used in accordance with this invention. Mutations are developed using conventional methods of directed mutagenesis, known to those of ordinary skill in the art. Moreover, the invention provides for functionally equivalent interferon-beta-1a polypeptides. A form of interferon beta-1a is currently commercially available from Biogen, Inc in the commercial name of AVONEX®. Another form of interferon beta-1a is currently commercially available from Serono, Inc in the commercial name of Rebif®.

The term "interferon-beta-Ib" or "IFN-β-1b" as used herein refers to a mutein of IFN-beta polypeptide having residue Cys17 replaced by residue Ser17, and expressed in a non-glycosylated form, with the N-terminal amino acid, methionine, post-translationally removed (See U. S. Patent No. 4,588, 585). Interferon beta-1b is therefore a purified, sterile, lyophilized protein product that may be produced by recombinant DNA technology. Interferon beta-1b may be manufactured by bacterial fermentation of a strain of Escherichia coli that bears a genetically engineered plasmid containing the gene for human interferon beta ser17 (see. Lin et al. (1986) and U. S. Patent No. 5,814, 485). Interferon beta-1b has 165 amino acids and an approximate molecular weight of 18.5 kD. A form of interferon beta-1b beta is currently commercially available from BERLEX (Richmond, California). in the commercial name of BETASERON®.

In another embodiment, the invention provides therefore a method for treating a polyglutamine expansion neurodegenerative disease comprising administering a subject in need thereof with a therapeutically effective amount of an inducer of PML expression as above described.

In the context of the invention, the term "treating" or "treatment", as used herein, means reversing, alleviating, inhibiting the progress of, or preventing the disorder or condition to which such term applies, or one or more symptoms of such disorder or condition.

According to the invention, the term "patient" or "patient in need thereof', is intended for a human or non-human mammal affected or likely to be affected with a polyglutamine expansion neurodegenerative disease. In a preferred embodiment, the patient may be diagnosed with such diseases but still be asymptomatic or with an early stage disease.

By a "therapeutically effective amount" of the inducer of PML expression as above described is meant a sufficient amount of inducer of PML expression to treat a polyglutamine expansion neurodegenerative disease at a reasonable benefit/risk ratio applicable to any medical treatment. It will be understood, however, that the total daily usage of the compounds and compositions of the present invention will be decided by the attending physician within the scope of sound medical judgment. The specific therapeutically effective dose level for any particular patient will depend upon a variety of factors including the disorder being treated and the severity of the disorder; activity of the specific compound employed; the specific composition employed, the age, body weight, general health, sex and diet of the patient; the time of administration, route of administration, and rate of excretion of the specific compound employed; the duration of the treatment; drugs used in combination or coincidential with the specific polypeptide employed; and like factors well known in the medical arts. For example, it is well within the skill of the art to start doses of the compound at levels lower than those required to achieve the desired therapeutic effect and to gradually increase the dosage until the desired effect is achieved. However, the daily dosage of the products may be varied over a wide range from 0.01 to 1,000 mg per adult per day. Preferably, the compositions contain 0.01, 0.05, 0.1, 0.5, 1.0, 2.5, 5.0, 10.0, 15.0, 25.0, 50.0, 100, 250 and 500 mg of the active ingredient for the symptomatic adjustment of the dosage to the patient to be treated. A medicament typically contains from about 0.01 mg to about 500 mg of the active ingredient, preferably from 1 mg to about 100 mg of the active ingredient. An effective amount of the drug is ordinarily supplied at a dosage level from 0.0002 mg/kg to about 20 mg/kg of body weight per day, especially from about 0.001 mg/kg to 7 mg/kg of body weight per day.

In another one embodiment the invention relates to the use of at least one nucleic acid encoding for an inducer of PML expression for the manufacture of a medicament intended for treating a polyglutamine expansion neurodegenerative disease. Such nucleic acids include those encoding for interferon polypeptides, and in particular for interferon-beta polypeptides as above described.

### Pharmaceutical compositions

The inducer of PML expression or nucleic acid encoding for such inducer of expression may be combined with pharmaceutically acceptable excipients, and optionally sustained-release matrices, such as biodegradable polymers, to form therapeutic compositions.

"Pharmaceutically" or "pharmaceutically acceptable" refers to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type.

In the pharmaceutical compositions of the present invention for oral, sublingual, subcutaneous, intramuscular, intravenous, transdermal, local or rectal administration, the active principle, alone or in combination with another active principle, can be administered in a unit administration form, as a mixture with conventional pharmaceutical supports, to animals and human beings. Suitable unit administration forms comprise oral-route forms such as tablets, gel capsules, powders, granules and oral suspensions or solutions, sublingual and buccal administration forms, aerosols, implants, subcutaneous, transdermal, topical, intraperitoneal, intramuscular, intravenous, subdermal, transdermal, intrathecal and intranasal administration forms and rectal administration forms.

Preferably, the pharmaceutical compositions contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

The pharmaceutical forms suitable for injectable use include sterile aqueous solutions or dispersions; formulations including sesame oil, peanut oil or aqueous propylene glycol ; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi.

Solutions comprising compounds of the invention as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

A inducer of PML expression used in the invention, and especially interferon polypeptides can be formulated into a composition in a neutral or salt form. Pharmaceutically acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like.

The inducer of PML expression may be combined with other active ingredients if desired.

The carrier can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin.

Sterile injectable solutions are prepared by incorporating the active polypeptides in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed.

For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 ml of isotonic NaCl solution and either added to 1000 ml of hypodermoclysis fluid or injected at the proposed site of infusion. Some variation in dosage will necessarily occur depending on the condition of the subject being treated. The person responsible for administration will, in any event, determine the appropriate dose for the individual subject.

The compounds of the invention may be formulated within a therapeutic mixture to comprise about 0.0001 to 1.0 milligrams, or about 0.001 to 0.1 milligrams, or about 0.1 to 1.0 or even about 10 milligrams per dose or so. Multiple doses can also be administered.

In addition to the compounds of the invention formulated for parenteral administration, such as intravenous or intramuscular injection, other pharmaceutically acceptable forms include, e.g. tablets or other solids for oral administration ; liposomal formulations ; time release capsules ; and any other form currently used.

A compound of the invention can be delivered in a pharmaceutically acceptable ophthalmic vehicle, such that the compound can penetrate the corneal and internal regions of the eye, as for example the anterior chamber, posterior chamber, vitreous body, aqueous humor, vitreous humor, cornea, iris/ciliary, lens, choroid/retina and sclera. The pharmaceutically-acceptable ophthalmic vehicle may, for example, be an ointment, vegetable oil or an encapsulating material. Alternatively, the compound of the invention may be injected directly into the vitreous, aqueous humour, ciliary body tissue(s) or cells and/or extra-ocular muscles by electroporation means.

The invention will further be illustrated in view of the following figures and examples.

### FIGURES :

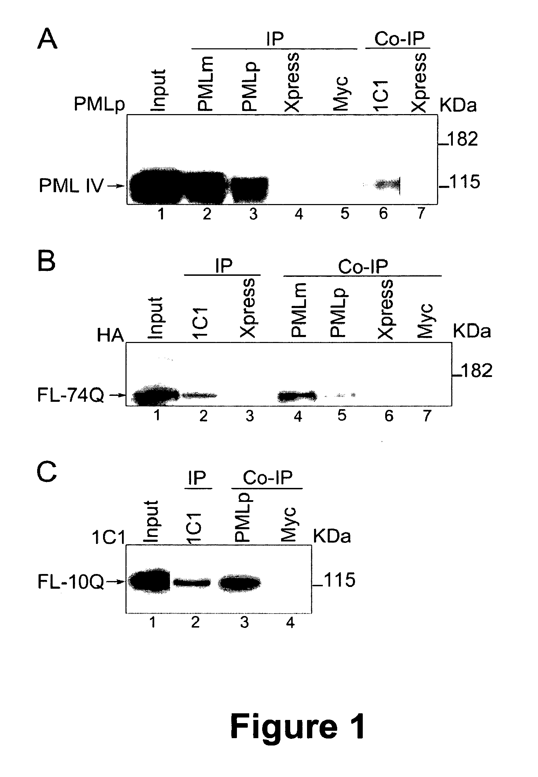

**Figure 1.** ATXN7 interacts with PML IV.
   A, B) Mutant (FL-74Q) or wild-type (FL-10Q) ATXN7 and PML IV were coexpressed in COS-7 cells and immunoprecipitated (IP) or co-immunoprecipitated (Co-IP). Antibodies used: 1C1 monoclonal anti ATXN7; PML m: PML monoclonal, PML p: PML polyclonal; anti X-press (monoclonal negative control); anti Myc (polyclonal negative control). A) IP of PML IV (lanes 2 and 3) and co-IP of PML IV with ATXN7 (lane 6); co-IP was not seen with control antibodies (lanes 4, 5 and 7). B) IP of mutant ATXN7 (FL-74Q) (lane 2) and co-IP with PML (lanes 4 and 5), co-IP was not seen with control antibodies (lanes 3, 6 and 7). C) IP of FL-10Q (lane 2) and co-IP with PML IV (lane 3); co-IP was not seen with the control antibody. Panels A, B and C: The lanes shown in each panel were on the same blots; cuts were made to eliminate lanes irrelevant to the demonstration.

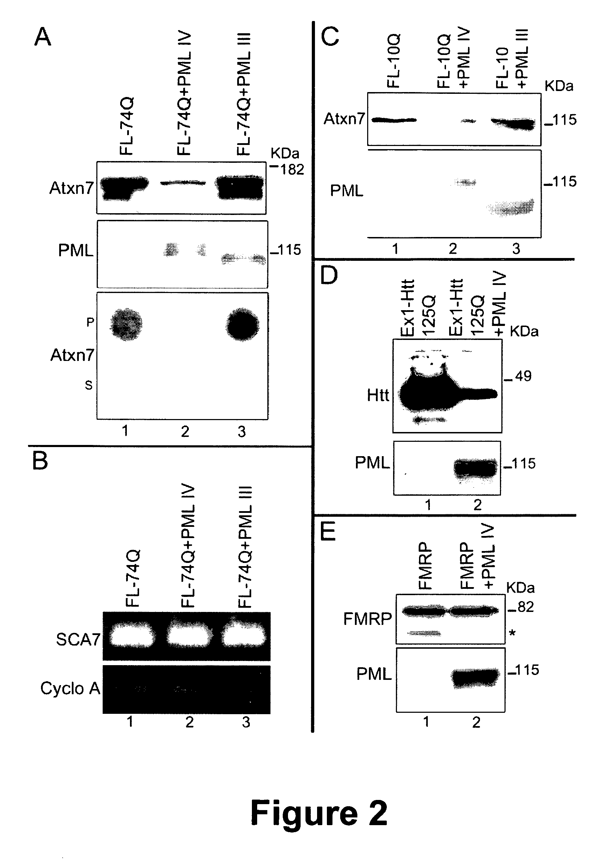

**Figure 2.** ATXN7 and Huntingtin exon1 are efficiently degraded in the presence of PML IV.
   A) Western blot and filter assay of FL-74Q alone or coexpressed with PML IV or III in COS-7 cells. Upper panels: Western blot (30 µg protein, anti ATXN7 IC1, anti PML p). ATXN7 levels strongly decreased in the presence of PML IV (lane 2) but slightly increased with PML III (lane 3). Bottom panel: Pellet (P) or supernatant (S) fractions of cell extracts (30 µg protein) were filtered and SDS-insoluble ATXN7 was revealed by anti ATXN7 1C1. Coexpression of PML IV led to the disappearance of SDS-insoluble aggregates of mutant ATXN7 (lane2), whereas PML III slightly increased ATXN7 (lane 3compared to lane 1). B) RT-PCR. mRNAs were extracted from cells expressing FL-74Q alone (lane 1) or combined with PML IV (lane 2) or III (lane 3). Primers specific to the SCA7 cDNA, and the cyclophilin A (Cyclo A) reference cDNA were used for PCR. Coexpression of PML IV or PML III had no effect on mutant ATXN7 mRNA. C) Western blot of cells expressing FL-10Q alone (lane 1) or coexpressed with PML IV (lane 2) or III (lane 3). PML IV decreased and PML III slightly increased ATXN7 levels (same antibodies as in panel A). D) Western blot of mutant Htt exon 1 expressed in HeLa cells. Htt exon1 (Ex1) with 125Q alone (lane 1) or with PML IV (lane 2): levels of Htt Ex1 (detected with antibody IC2) were strongly decreased when PML IV was coexpressed. E) Western blot of FMRP expressed in COS-7 cells alone (lane 1) or with PML IV (lane 2): PML IV did not modify the amount overexpressed FMRP (detected with antibody IC3). * Unknown protein degraded in the presence of PML IV. In panels C, D and E, the anti PML antibody was as in panel A.

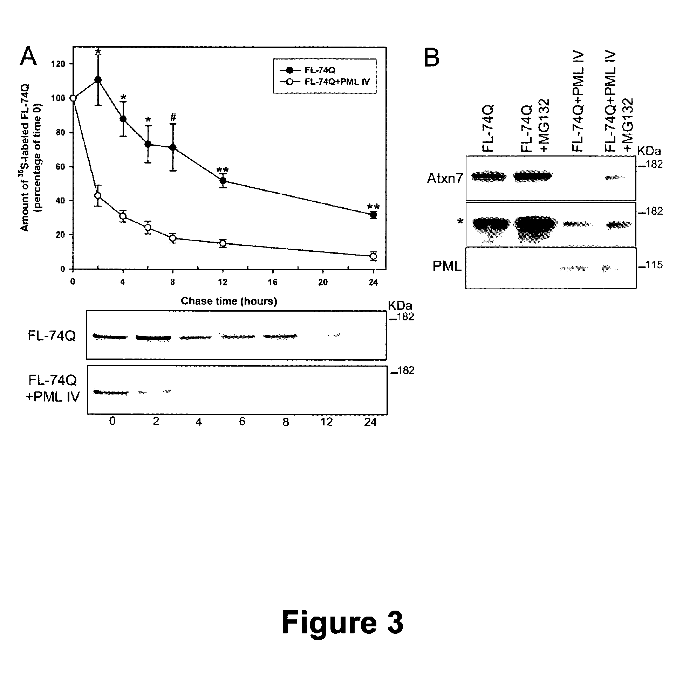

**Figure 3.** PML IV promotes the degradation of mutant ATXN7 by proteasomes.
   A) Pulse-chase analysis of the turnover of FL-74Q expressed alone or with PML IV in COS-7 cells. Quantification of immunoprecipated ATXN7 from four independent experiments are expressed as means +/- SEM. Symbols (*, #) above each time point indicate statistical significant differences between FL-74Q and FL-74Q+PML IV (Paired t-test: * P£ 0.002; **P£ 0.001; Mann Whitney test: # P£ 0.03).
   B) Inhibition of the proteasome by MG132 (5µM) treatment in COS-7 cells significantly increases the amount of FL-74Q expressed alone (lane 2, compared to lane 1) or with PML IV (lane 4 compared to lane 3). *Longer exposure of same blot. PML IV levels in bottom panel.

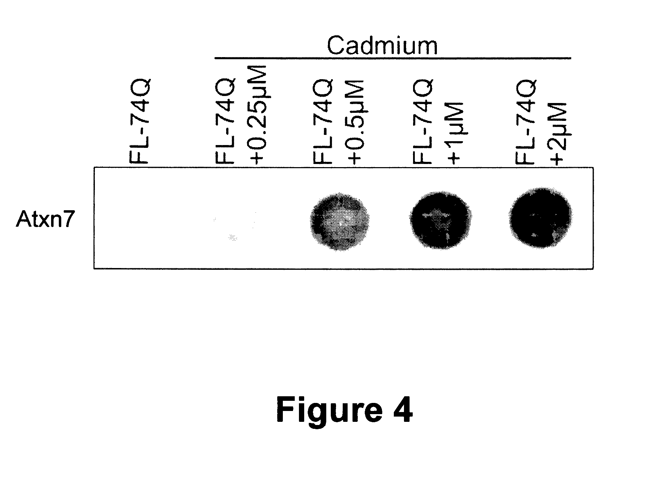

**Figure 4**. Cadmium disrupts PML bodies and enhances aggregation of mutant ATXN7.
   The increase in ATXN7 aggregation is dose-dependent in cells treated with 0.25 µm to 2 µM cadmium (filter assay on pellet fraction of cell extracts (50 µg).
**Figure 5.** Interferon-beta treatment leads to the degradation of mutant ATXN7 and the disappearance of aggregates.
   B) RT-PCR with primers specific to PML IV shows that ß-INF increases the expression of endogenous PML IV mRNA in non transfected (NT) cells (compare lane 2 to lane 1), the levels of which are compared to overexpressed PML IV (positive control, lane 3) and overexpressed PML III (negative control, lane 4). The reference transcript cyclophilin A (CycloA) was amplifed in parallel. C-E) ß-INF induces the expression of multiple isoforms of PML (C-E). ß-INF does not affect the level of endogenous ATXN7 (C), but decreases the levels of transfected wild-type (D) and mutant ATXN7 (E). F) PML-dependent degradation of ATXN7 induced by ß-INF is inhibited by MG132, showing that it is mediated by the proteasome. G) Filter assay shows in the left panel that ATXN7 aggregates disappear in ß-INF-treated cells, but an insoluble PML-containing fraction appears (lane 2). The right panel shows that insoluble PML material was observed either when PML IV was expressed (lane 3), or when NT cells were treated with ß-INF (lane 2), but not when PML III was expressed (lane 4).

### EXAMPLE:

### Materials and methods

**Plasmids :** The SCA7 constructs were previously described (Zander et al, 2001). FL-10Q and FL74Q, cloned in pCDNA3, contain the full-length (FL) wild-type or expanded ATXN7 cDNA, respectively, with FLAG tags at the C-terminus. FL-74Q contains, in addition, an HA tag at the N-terminus. Full-length and truncated ATXN7 containing 100Q were cloned in the pEGFP-N1 vector.

Construct PML IV was generated by amplifying PML IV cDNA sequence by PCR using primers to introduce a Kozak sequence and the BamH1 site just upstream of PML IV ATG and the Xbal restriction site after the stop codon of PML IV. The PCR product was then digested with BamH1 and Xbal and cloned into pCDNA3 at the corresponding sites. PML IV was then cloned in pCDNA3. HcRFP-PML IV plasmid was generated by subcloning PML IV from pCDNA3 into pHcRed1-C1 vector (Clontech). All PML constructions were verified by sequencing.

**RNA preparation and semi-quantitative RT-PCR : RNA was extracted from** whole cell lysates of transfected or untransfected COS-7 cells, using the RNAeasy kit (Quiagen), and cDNAs were synthesized (ThermoScript RT-PCR System, Invitrogen) according to the manufacturers' instructions. PCR was performed using primers specific for the genes of interest. Primers used for specific detection of PML IV were located in exon 8, and the reverse primer was designed to anneal only PML IV mRNAs. The cyclophilin A gene was used as the reference.

**Cell culture, transfections, MG132, INF and cadmium treatments :** COS-7 cells were maintained in DMEM (Invitrogen) supplemented with 5% FBS, and penicillin-streptomycin (100 Ul/ml; 100 µg/ml). Cell lines were transfected with Lipofectamine-PLUS reagents (Invitrogen) as prescribed. Unless otherwise specified, cells were harvested or analyzed by immunofluorescence 40-45 h post-transfection. ß-INF. Recombinant human Interferon beta (Hu-IFN-b) is obtained from human fibroblast mRNA expressed in E. Coli. Its molecular weight is approximately 20 kDa. Recombinant human Interferon beta was supplied as a solution at 1×106 units/ml in PBS (phosphate buffered saline) containing 0.1% BSA (bovine serum albumin) and 10% glycerol. It was purchased from Sigma-Aldrich. Recombinant human Interferon beta (2000 Ul/ml) was added to the culture medium after plating and during transfection. Cadmium chloride (Cadmium chloride hemi pentahydrate, Sigma Aldrich) (from 0.25 µM to 2 µM) was added during transfection. For proteasome inhibition, 5 µM MG132 was applied for 12 h, 25 h post-transfection. Cultures of post-mitotic neurons were prepared from the cortex of embryonic day 16 Wistar rats. Cells were extracted from the embryos and cultured with 1 µM MK801. Glial cell proliferation was stopped by adding 1 µM cytosine arabinoside after 3 h. Primary cultures of cortical neurons were transfected 96 h after plating using Lipofectamine 2000 reagent (Invitrogen) according to the manufacturer's instructions.

**Western blot and filter retardation assay** : Cells were harvested in PBS, pelleted and lysed 30 min on ice in lysis buffer containing 50 mM Tris pH 8.8, 100 mM NaCl, 5 mM MgC12, 0.5% NP40, 1 mM EDTA and 250 Ul/ml benzonase (Merck) supplemented with a cocktail of protease inhibitors (Complete and Pefabloc, Roche). Total extracts were centrifuged at 13000 rpm for 10 min at 4°C to separate soluble proteins from aggregates and membranes. Supernatants (30 µg protein) were analyzed by Western blot. Pellets were washed with PBS and further incubated 30 min on ice in a pellet buffer containing 20 mM Tris pH 8.0, 15 mM MgCl2 and 250 Ul/ml benzonase. Protein concentrations (supernatant and pellet) were determined by Bio-Rad assay. Samples were prepared for Western blot and for filter assay as previously described (Sittler et al., 1998; Wanker et al., 1999).

**Pulse-chase analysis of ATXN7 turnover** : Pulse-chase experiments were performed 17 h after transfection of COS-7 cells with FL-74Q or with FL-74Q and PML IV. Cells were starved in methionine-cysteine-free DMEM containing 2% FBS, for 1 h. Cells were labeled for 1 h with 200 µCi/ml of Pro-mix L-35S labeling mix (Amersham) in starvation medium. After the pulse, cells were rinsed twice and chased for the indicated periods of time in DMEM with 5% FBS. For immunoprecipitation, cells were lysed in 50 mM Tris pH 8.0, 300 mM NaCl, 1% NP40, 5% glycerol and protease inhibitors. Equal amounts of labeled lysates (12x 106 c.p.m. at time 0, determined after TCA precipitation) were immunoprecipitated with 3 µg anti HA antibody (coupled to protein G sepharose beads) for 2h at 4°C. Beads were washed 4 times with RIPA buffer and bound proteins were directly denatured in 40 µl Laemmli buffer. Immunoprecipitates were resolved on 4-12% SDS gradient gels (Invitrogen), visualized by phosphoimaging and quantified with Aida analysis software.

**Immunofluorescence:** Cells plated on glass coverslips coated with polylysine (COS-7) or polyethyleneimine (cortical neurons) were fixed for 20 min with 4% paraformaldehyde. Primary neurons were fixed at 4 days post-transfection. Permeabilization and further incubations with antibodies were as previously described (Sittler et al., 1998). Cells were mounted with Fluoromount-G and samples were observed with a Leica SP1 confocal microscope equipped with a 63X/1.32 numerical aperture (N. A.) objective. Leica confocal software was used to acquire images. For conventional analysis on a Zeiss Axioplan-2 microscope equipped with 25X/ N. A., 63X/1.32 N. A. and 100X/ N. A. objectives, Explora Nova software was used for image aquisition. Cryostat sections (9 µm) of fixed retina were prepared and used for immunofluorescence detection as described (Yvert et al., 2000).

**Antibodies :** For Western blot (WB) and immunofluorescence (IF) analysis, we used the following antibodies and concentrations: 1C1 mouse anti ATXN7 (Yvert et al., 2000) at 1:5000 (WB) and 1:1000 (IF), mouse anti HA (Babco) at 1:10000 (WB) and 1:4000 (IF), rabbit anti ATXN7 (Affinity BioReagents) at 1:500 (IF), rabbit anti ATXN7 affinity-purified polyclonal antibody 1261 at 1:100 (IF on fixed retina only), mouse anti PML (PG-M3, Santa Cruz) at 1:500 (WB and IF), rabbit anti PML (H-238, Santa Cruz) at 1:500 (WB and IF), chicken anti PML antibody at 1:500 (IF on fixed retina only), 1 C2 anti polyQ monoclonal antibody at 1:2000 (WB), anti FMRP 1 C3 antibody (Sittler et al., 1996) at 1:1000, mouse anti poly-ubiquitinylated proteins (FK2, Biomol) at 1:1000 (IF), rabbit anti 19S proteasome subunit S10a (PW-8225, Biomol) at 1:500 (IF), rabbit anti 20S core (PW-8155, Biomol) at 1:1000 (IF), rabbit anti Hsp40 (SPA-400, Stressgen) at 1:2000 (IF). For the filter assay, we used the same antibody concentrations as for Western blots. For immunofluorescence, secondary antibodies were: Alexa 488-conjugated goat anti mouse or goat anti rabbit IgG (Molecular Probes) used at 1:500 and CY3-conjugated goat anti mouse or goat anti rabbit IgG (Jackson Immunoresearch) used at 1:1000. Different secondary antibodies and dilutions were used: CY3-conjugated goat anti rabbit IgG (1:200) and FITC-conjugated goat anti chicken IgG (1:150). For immunofluorescence (IF) analysis, we used the following antibodies and concentrations: rabbit anti 19S proteasome subunit S5a (PW-9250, Biomol),mouse monoclonal anti 19S proteasome subunit S6b (TBP7) (PW-8765, Biomol), mouse monoclonal anti 20S alpha 2 proteasome subunit (PW-8105, Biomol).

**Time-lapse recordings** : HeLa Kyoto cells grown on 35 mm microwell dishes (MatTek Corporation) were transfected with 1 µg FL-100Q-EGFP (expressing GFP-ATXN7 fusion protein) and 0.5 µg HcRFP-PML IV using Fugene (Roche). At 6 h post-transfection, cells were placed on the microscope in a chamber at 37°C and 5% CO2. Several cells showing low levels of GFP-ATXN7 were selected and monitored every 10 min for 16 h. Images were acquired with a Leica DMIRE2 inverted microscope equipped with a 63X/1.35 numerical aperture objective and a Photometrics Cool Snap FX camera. Metamorph software was used to control image acquisition and manipulation.

**Co-immunoprecipitation :** COS-7 cells were transfected in 90 mm culture dishes with PML IV and FL-74Q or FL-10Q. Cells were harvested 42-45 h post transfection and lysed on ice in 150 mM salt buffer (50 mM Tris pH 8.0, 150 mM NaCl, 1 mM EDTA, 1% NP40) supplemented with a cocktail of protease inhibitors (Complete, Pefablock). Total extract was centrifuged at 13000 rpm for 10 min at 4°C and the supernatant was pre-cleared with sepharose A/G beads for 1 h at 4°C. Pre-cleared supernatant and protein G or A sepharose beads (Amersham Biosciences) coupled with the appropriate antibodies were incubated 2 h at 4°C on a rotating wheel for immunoprecipitation. Finally, the beads were washed 5 times with 500 mM salt buffer (50 mM Tris pH 8.0, 500 mM NaCl, 1 mM EDTA, 1% NP40; protease inhibitors) and bound proteins were directly denatured by heating for 5 min at 95°C in 30 µl Laemmli buffer. Samples were analyzed by Western blot.

**Electron microscopy:** Cells were fixed with 4% paraformaldehyde in phosphate buffer pH 7.4 for 1 h on ice, then post-fixed with 1% osmium tetroxyde and embedded in Epon 812 (Taab). Ultrathin sections (50 nm), placed on nickel grids, were immunogold-labeled after embedding. We used primary antibodies mouse monoclonal anti ATXN7 1C1 at 1:200 and rabbit polyclonal anti PML at 1:250 and secondary antibodies conjugated to 10 or 15 nm gold particles (goat anti mouse or goat anti rabbit (Aurion) at 1:100. After labeling, sections were post-fixed in 1 % glutaraldehyde and stained with uranyl acetate and lead citrate. Following embedding, ultrathin sections of 50 nm were placed on nickel grids and the following protocol was used. The sections were treated with 1 % periodic acid for 5 min, washed in distilled water, treated with sodium metaperiodate and washed. Samples were incubated in BSA 3% in TBS pH 7.6 for 40 min, followed by incubation in primary antibodies (mouse monoclonal anti ATXN7 1C1 at 1:200 and rabbit polyclonal anti PML at 1:250) in TBS pH 7.6, overnight at 4°C. The samples were washed in TBS buffer (pH 7.6 and pH 8.2), followed by incubation with secondary antibodies conjugated to 10 or 15 nm gold particles (goat anti mouse or goat anti rabbit (Aurion) 1:100 in TBS pH 8.2) for 1 h at room temperature. After washes in TBS pH 8.2, sections were post-fixed in 1 % glutaraldehyde for 10 min and rinsed in distilled water before staining with uranyl acetate for 15 min and lead citrate for 7 min. Cells were examined with a Jeol 1200EX electron microscope at 80 Kvolts.

### Results

**Aggregated ATXN7 colocalizes with PML bodies in SCA7 transgenic mice :** We previously showed that, PML bodies colocalized with a subset of polyQ aggregates in the brains of patients with SCA7 (Takahashi et al., 2002). To determine whether this was also the case in models of SCA7, we examined the brains of transgenic mice B7E2.B, which express mutant ATXN7 with 128 glutamines ubiquitously in the CNS and develop progressive ataxia (Yvert et al., 2001). Mutant ATXN7 accumulated in most neurons, including the cerebellar Purkinje cells and retinal neurons, leading to NI formation. A subset of large ATXN7 aggregates in Purkinje cells and retinal ganglion neurons contained PML bodies. Ganglion neurons also had small ATXN7 aggregates, which were either sparsely distributed in the nucleus or juxtaposed to large aggregates. PML bodies colocalized with small aggregates, in some cases forming a ring around the aggregate. Some PML bodies were juxtaposed to both small and large aggregates. Colocalization of PML bodies with ATXN7 aggregates, notably small ones, is consistent with our previous study of SCA7 brains, and raises the question of how PML bodies affect the aggregation of mutant ATXN7.

**ATXN7 colocalizes with PML bodies in cell culture models :** To investigate the relationship between PML bodies and mutant ATXN7, we coexpressed full-length wild-type and mutant ATXN7 with isoforms of PML in neurons and in COS-7 cells. There are seven isoforms of PML, resulting from differential splicing, that form nuclear bodies with different morphologies (Beech et al., 2005; Bischof et al., 2002). We have examined the effect of exogenous expression of PML IV that was previously shown to induce cellular senescence, apoptosis, and regulate p53 activity (Bischof et al., 2002; Fogal et al., 2000), and may thus be related to neuronal dysfunction or death. The effects of PML III were examined to control for specificity.

Endogenous ATXN7 was distributed homogeneously in a granular pattern throughout the nucleus, and did not colocalize with the small endogenous nuclear bodies observed in neurons. When PML IV was overexpressed in transfected neurons, both endogenous ATXN7 and full-length wild-type (FL-10Q) exogenous ATXN7 colocalized with PML IV in larger nuclear bodies. In COS-7 cells, mutant ATXN7 with 74Q (FL-74Q), was also distributed homogeneously in the nucleoplasm of the majority of transfected cells when expressed alone (not shown). In 10-20% of the cells, however, multiple dense nuclear aggregates were seen that contained endogenous PML bodies in their center, as well as the Daxx protein, a resident PML body protein (not shown). Exogenous PML III formed rod-shaped bodies and exogenous PML IV larger patchy structures, some of which had a ring-like shape (not shown). When PML III was coexpressed with FL-74Q, both rod-shaped PML III bodies and ATXN7 aggregates were observed, but they rarely colocalized. In contrast, PML IV and mutant ATXN7 colocalized in large, rounder nuclear structures, that differed from the dense ATXN7 aggregates, but resembled the PML IV bodies, in that some had a ring-like shape (. In primary cultures of rat cortical neurons, PML IV also formed round nuclear bodies, but they were smaller and morphologically more homogenous than in COS-7 cells. When mutant ATXN7 and PML IV were coexpressed in these neurons, mutant ATXN7 was localized in the PML IV bodies. These data suggest that PML IV bodies recruit both endogenous ATXN7 and exogenous normal and mutant ATNX7.

**PML IV recruits soluble mutant ATXN7 to nuclear bodies :** To determine whether PML IV recruits ATXN7 to the nuclear bodies, we performed video-recorded time-lapse experiments on living HeLa Kyoto cells expressing full length ATXN7 with 100Q fused to EGFP (EGFP-FL-100Q) and PML IV fused to RFP. The cells were monitored from 6 h post-transfection to 22 h post-transfection (i.e. for 16 h). In cells expressing EGFP-FL-100Q alone, the intensity of the green fluorescence increased over time up to 22 h post-transfection but remained homogeneously distributed in the nucleoplasm (not shown), indicating that endogenous PML bodies do not form ATXN7-positive bodies. In the presence of RFP-PML IV (red), EGFP-FL-100Q fluorescence remained weak in the nucleoplasm, but increased in PML IV bodies from the moment they formed, indicating that the soluble form of mutant ATXN7 was recruited to the PML IV bodies. Small ATXN7-containing PML IV bodies then moved and coalesced into larger bodies. PML IV, therefore, recruits soluble mutant ATXN7 to nascent PML IV bodies, preventing its accumulation in the nucleus and formation of focal aggregates. Coalescence of small bodies into larger ones was also observed in cells expressing RFP-PML IV alone (not shown).

**ATXN7 interacts with PML IV:** To determine whether recruitment of ATXN7 to PML IV bodies was due to a physical interaction with PML IV in cell nuclei, we performed co-immunoprecipitation experiments. We first verified that two different anti-PML antibodies (PMLm and PMLp) immunoprecipitated PML IV (Fig. 1 A, lanes 2 and 3) and that the anti-ATXN7 monoclonal antibody 1C1 immunoprecipitated FL-10Q (Fig. 1 C, lane 2) and FL-74Q (Fig. 1 B, lane 2). The control antibodies (anti-myc and anti-Xpress) did not immunoprecipitate either protein (Fig. 1 A, lanes 4,5 and 7; Fig. 1 B, lanes 3, 6 and 7). When HA tagged FL-74Q and PML IV were coexpressed and ATXN7 was immunoprecitated with 1C1, PML IV co-precipitated in the complex (Fig. 1 A, lane 6). When PML IV was immunoprecipitated, HA tagged FL-74Q co-precipitated in the complex (Fig. 1 B, lane 4 and 5). FL-10Q and PML IV also co-immunoprecipitated (Fig. 1 C, lane 3). Therefore, not only mutant full length ATXN7 but also its wild-type counterpart physically interact, directly or indirectly, with PML IV in nuclear bodies.

### PML IV bodies degrade mutant ATXN7 and prevent aggregate formation:

We then determined the effect of PML IV on the biochemical properties of mutant ATXN7. When FL-74Q alone was expressed in COS-7 cells, both soluble and aggregated ATXN7 were detected. The soluble form of mutant ATXN7 appeared as a 150 kDa protein on Western blots (Fig. 2 A, top). The aggregated form of the protein was retained on cellulose acetate filters in the filter retardation assay (Fig. 2A, bottom) that evaluates the overall amount of SDS-insoluble amyloid fibers formed by aggregation of proteins with expanded polyQ (Wanker et al., 1999). Aggregated ATXN7 was found in the pellet when cell homogenates were centrifuged, but not in the supernatant. Coexpression with PML III very slightly increased the amounts of soluble and aggregated ATXN7 (Fig. 2A, top and bottom). In contrast, coexpression with PML IV led to a strong decrease in the amounts of soluble and aggregated ATXN7 (Fig. 2 A, top and bottom). The supernatant obtained after centrifugation of cell homogenates was also devoid of SDS-insoluble ATXN7. The difference between the effects of PML III and IV was not due to different levels of expression of the isoforms (Fig. 2 A, top), nor to differential effects on the transcription of the ATXN7 gene (Fig. 2 B). This suggests that PML IV bodies act directly on the ATXN7 protein, by increasing its degradation.

We then examined whether PML IV-dependent degradation of ATXN7 depends on the length of the polyQ tract. When expressed with PML IV, the level of wild-type FL-10Q also decreased (Fig. 2 C), whereas its level slightly increased when expressed in the presence of PML III. Degradation therefore does not depend on the presence of an expanded polyQ tract.

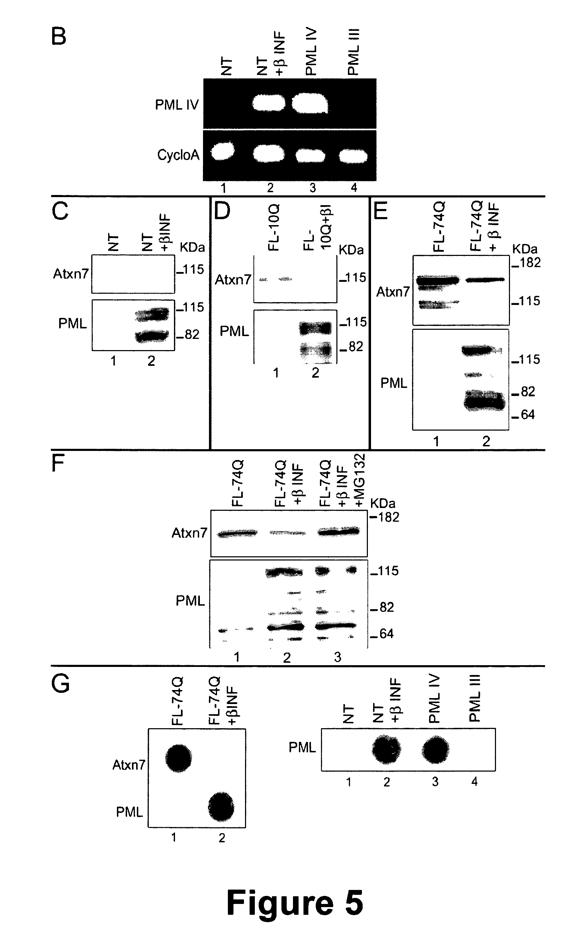

To determine whether PML IV bodies also degrade other polyQ-containing proteins, we coexpressed PML IV with huntingtin (htt)-exon1 harboring 125Q. PML IV strongly reduced the level of soluble mutant htt-exon1 (Fig. 2 D). We also verified whether PML IV would degrade an unrelated nuclear protein FMRP (Fragile X Mental Retardation Protein) (Sittler et al., 1996) without a polyQ sequence. The level of expression of exogenous FMRP was unaffected by coexpression with PML IV (Fig 5 E). Intriguingly, a second faint band of lower molecular weight appeared to be decreased by PML IV. Its identity is unknown.

These results show that PML IV bodies recruit both exogenous mutant and normal polyQ-containing proteins and prevent their accumulation in the nucleus, but have no effect on another exogenous nuclear protein, FMRP, showing that PML IV targets were not degraded because they were mutated or overexpressed. The targets of PML IV are therefore selective, although the basis for the selectivity is not known.

**PML IV promotes the degradation of mutant ATXN7 by proteasomes :** To determine whether PML IV increased the rate of degradation of mutant ATXN7 we performed pulse-chase experiments in COS-7 cells in which mutant ATXN7 was expressed either alone or in combination with PML IV. The degradation of mutant ATXN7 expressed alone was relatively slow, in good agreement with a previously published pulse-chase experiment (Yvert et al., 2001). During the first 8 h of the chase period, only 28% of ATXN7 was degraded (Fig. 3A). In the presence of PML IV, however, 83% of mutant ATXN7 was degraded after 8 h (Fig. 3A). The difference between the levels of ATXN7 in the presence and in the absence of PML IV was significant at all time points, starting at 2 hr, indicating that PML IV bodies accelerate the degradation of mutant ATXN7.

To see whether the degradation of mutant ATXN7 in the presence of PML IV was mediated by proteasomes, COS-7 cells expressing FL-74Q in the presence and in the absence of PML IV were treated with the proteasome inhibitor MG132 (5µM). Inhibition of the proteasome increased the amount of mutant ATXN7 in the cells coexpressing FL-74Q and PML IV (Fig. 3 B, lane 4 compared to lane 3). However, since PML IV was already active before MG132 treatment, it was not possible to accumulate ATXN7 to the level observed without PML. MG132 also increased the level of mutant ATXN7 expressed in the absence of PML IV, by impeding normal turnover of the protein (Fig. 3 B, lane 2 compared to lane 1). We thus conclude that the effect of PML IV on the degradation of ATXN7 was mediated by the ubiquitin-proteasome system.

### PML IV bodies recruit UPS components to degrade mutant ATXN7 :

Under certain stress conditions, PML forms clastosomes that contain components of the UPS (Lafarga et al., 2002). To determine whether PML IV bodies are clastosomes and to understand how they may clear ATXN7, they were analyzed for the presence of UPS components. S10a, a subunit of the 19S non-ATPase regulatory complex of the proteasome was not found in focal aggregates formed by mutant ATXN7, but was highly colocalized with PML IV bodies when only PML IV was expressed. Interestingly, when coexpressed with PML IV, mutant ATXN7 colocalized perfectly with the S10a subunit in PML IV bodies. Similar observations were made with two other 19S proteasome subunits, S5a and S6b; the latter was already detected in endogenous clastosomes (Lafarga et al., 2002). The 20S catalytic core of the proteasome colocalized only with a subset of aggregates in cells expressing FL-74Q alone but colocalization was complete with PML IV bodies containing the mutant protein, as well as with PML IV expressed alone. This was also observed for subunit a2 of the 20S proteasome. The same was true for the chaperone Hsp40, that recognizes misfolded polyQ proteins. We next verified whether ATXN7 was ubiquitinylated in PML IV bodies. Non-transfected cells possessed only a few PML bodies labeled with an antibody against polyubiquitin that were possibly endogenous clastosomes. Aggregates of ATXN7, when expressed alone, were only slightly labeled with the anti-polyubiquitin antibody, but ATXN7- containing PML IV bodies were strongly labeled. Together, these data indicate that PML IV induces the assembly of a nuclear structure dedicated to protein degradation, reminiscent of clastosomes. They recruit both chaperones and proteasomes together with its substrates, such as mutant ATXN7, targeted for degradation by polyubiquitin.

**Recruitment of mutant ATXN7 in PML IV bodies prevents polyQ amyloid fiber formation :** We then investigated the nature of mutant ATXN7 in PML IV bodies by performing an ultrastructural analysis of transfected COS-7 cells. Since truncated mutant ATXN7 aggregates more rapidly than the full length protein (not shown), we expressed Tr-100Q-EGFP (the first 232 amino acids of the protein containing the polyQ stretch fused to EGFP) in the presence or absence of PML IV. We examined ATXN7 distribution by electron microscopy after immunolabelling with a monoclonal anti-ATXN7 antibody and a secondary antibody coupled with 10 nm gold particles. Tr-100Q-EGFP, when expressed alone, was restricted to pale-stained fibrillary structures in the nucleus with fibers radiating from the edges of the aggregates. When co-expressed with PML IV, Tr-100Q-EGFP colocalized with PML IV (co-labeling with species-specific secondary antibodies coupled with 15 nm gold particles to detect PML) in round or oval-shaped structures which are most likely PML IV bodies. Importantly, no fibrillary structures were evident in this case. This was confirmed by the absence of ATXN7 aggregates on the filter assay (Fig. 8 E). ATXN7 in PML IV bodies was therefore no longer fibrillary in structure and can no longer be considered to be aggregated. Soluble Tr-100Q-EGFP levels analyzed on Western blot were slightly decreased by PML IV expression (Fig. 8 E).

**Cadmium disrupts PML bodies and prevents the degradation of mutant ATXN7 by endogenous clastosomes :** To determine whether altering the amount of endogenous PML bodies affects the degradation or aggregation of mutant ATXN7, we treated cells expressing FL-74Q with cadmium chloride, reported to disrupt PML bodies, (Nefkens et al., 2003). Immunofluorescence analysis showed that 2 µM cadmium totally disrupted the endogenous PML bodies. Consequently no colocalization between mutant ATXN7 aggregates and the PML protein was observed, compared to the untreated cells. A treatment with increasing concentrations of cadmium (0.25 to 2 µM) showed that the aggregation of mutant ATXN7 was dose-dependent, as shown on filter assay (Fig. 9 B), suggesting that endogenous clastosomes are indeed involved in mutant ATXN7 degradation.

**Interferon-beta stimulates the PML-dependent degradation of mutant ATXN7 and the disappearance of aggregates :** Since interferon-beta (ß-INF) was reported to upregulate the expression of PML (Regad and Chelbi-Alix, 2001), we investigated its effect on the PML-dependent degradation of mutant ATXN7. ß-INF increased the size and number of PML bodies in non-transfected COS-7 cells. Faint endogenous PML-immunoreactivity was observed in aggregates of mutant ATXN7 in untreated cells. ß-INF treatment led to the formation of large strongly immunoreactive PML bodies that colocalized with mutant ATXN7. These results also suggest that the presence of mutant ATXN7 promoted the coalescence and fusion of PML bodies into enlarged bodies resembling PML IV bodies, which colocalized perfectly with mutant ATXN7. Semi-quantitative RT-PCR performed with primers that specifically amplify PML IV, showed that the expression of PML IV was induced in f3-INF-treated cells but not in control cells (Fig. 5 B, lane 2). Western blots show that ß-INF increased the levels of several PML isoforms in COS-7 cells expressing endogenous ATXN7, or exogenous ATXN7 in its wild type FL-10Q or mutant form FL-74Q (Fig. 5 C-E). ß-INF treatment decreased the amount of overexpressed soluble FL-10Q (Fig. 5 D) and FL-74Q (Fig. 5 E), but had no effect on the level of endogenous wild type ATXN7 (Fig. 5 C). The PML-dependent degradation of ATXN7 induced by ß-INF was mediated by the proteasome, as it was inhibited by MG132 (Fig. 5 F). Interestingly, insoluble mutant ATXN7 disappeared from cells treated with ß-INF, as shown by the filter assay, but an insoluble PML-containing material was detected (Fig. 5 G, left panel lane 2). This material was independent of ATXN7 expression, but dependent on ß-INF treatment and attributable to the increased expression of PML IV isoform (Fig. 5 G, right panel lanes 2 and 3).

We conclude from these experiments that ß-INF induces PML IV expression and increases the presence of clastosomes that degrade ATXN7, preventing its aggregation. The observation that only mutant but not endogenous ATXN7 is degraded in the presence of ß-INF suggests that this substance may have therapeutic value.

### Discussion

Accumulation of misfolded polyQ expansion proteins in neuronal nuclei, ultimately leading to the formation of NI, is a key step in the pathogenesis of SCA7 and other polyQ expansion diseases (Michalik and Van Broeckhoven, 2003). We have focused on PML bodies, known to be associated with NIs (Takahashi et al., 2003), that are suggested to be privileged sites of nuclear protein degradation (Lafarga et al., 2002), and have investigated their role in either the degradation or the accumulation of polyQ-containing protein. We report that modifying the level and composition of PML nuclear bodies has profound effects on the fate of disease-causing polyQ expansion proteins in the nucleus.

Seven PML isoforms have been described that share an N-terminal region comprising the RBCC (RING-finger, B-box, Coiled-Coil domain), but differ in their C-terminal region due to alternative splicing (Jensen et al., 2001). The role of each isoform is not yet known, but they might be associated with different functions of the nuclear bodies (Beech et al., 2005; Bischof et al., 2002; Muratani et al., 2002). We have explored the effect of two isoforms, PML III and IV, on the fate of mutant ATXN7 in the nucleus. Although these isoforms differ only by their short C-terminal extensions (71 aa in PML III and and 63 aa in PML IV), they form nuclear bodies that have strikingly different morphologies and protein composition. PML IV bodies are round and larger than PML III bodies which are rod-shaped. PML IV bodies were highly enriched in proteasome components. They contained subunits of the 19S complex, implicated in unfolding of the substrates, as well as subunits of the 20S catalytic core complex. PML IV bodies also colocalised with the chaperone Hsp40 and poly-ubiquitinylated proteins, and thus resemble clastosomes, a subset of endogenous PML bodies enriched in UPS components, suggested to be sites where nuclear proteins are degraded (Lafarga et al., 2002). Thus, endogenous PML IV, appear to be a component of endogenous clastosomes. Whether they also contain other isoforms of PML remains to be determined.

Interestingly, only overexpressed PML IV relocalised endogenous as well as exogenous wild type and mutant ATXN7 to PML IV bodies. Consequently, due to the presence of UPS components, PML IV actively increased the degradation of the soluble form of mutant ATXN7, which led to the disappearance of SDS-insoluble aggregates. We further demonstrated that the PML IV-dependent degradation of mutant ATXN7 is mediated by the proteasome, showing for the first time that exogenously expressed PML IV clastosomes indeed degrade a substrate, in our case mutant ATXN7.

Time-lapse experiments showed that PML IV acts on the soluble form of mutant ATXN7 to recruit it to PML IV bodies. Correspondingly, soluble PML IV physically interacts with the soluble forms of mutant and wild type ATXN7. This suggests that PML IV convoys the soluble forms of ATXN7 to the bodies, where they are degraded by the UPS components, thus preventing the deleterious accumulation of mutant ATXN7. How PML IV selects the substrates for degradation remains to be elucidated. Interestingly, ATXN7 does not interact with PML III (A.S. and A.J. unpublished), and consequently, it is not recruited to PML III bodies. However, the immediate early gene X1 (IEX-1), a stress response gene involved in apoptosis, was shown to colocalize with both PML III and IV and to co-immunoprecipitate with PML III, but not PML IV (Kruse et al., 2005). These authors noted on Western blots positive co-regulation of PML III with IEX-1 but a negative co-regulation of PML IV. IEX-1 might therefore be another nuclear substrate for PML IV clastosomes. An investigation of how ATXN7 interacts with PML IV, directly or indirectly, might provide a clue as to how PML IV selects its substrates.

The SDS-insoluble structures formed by PML IV, consistent with the fact that PML can self-interact and oligomerize (Grignani et al., 1996; Jensen et al., 2001; Kastner et al., 1992), might serve as a scaffold to bring together both the actors of the UPS and the substrates to be degraded. When the level of PML IV is increased by exogenous expression, the composition of the PML bodies changes, leading to the formation of enlarged clastosomes. This increases the capacity of the nucleus to degrade specific substrates, such as toxic polyQ protein. Others have shown that proteasome-dependent protein degradation occurs in nucleoplasmic foci that partially overlap or juxtapose with splicing speckles and PML bodies (Rockel et al., 2005). In a very recent study, a novel GTPase CRAG induced, under cellular stress, an interaction with PML that then formed the enlarged ring structures typical of PML bodies (Qin et al., 2006). Interestingly, it was shown that polyQ accumulation and oxydative stress triggered association of CRAG and polyQ in the cytoplasm before translocation to the nucleus. Once inside the nucleus, CRAG promoted the degradation of polyQ in PML bodies. It would be interesting to know whether CRAG was also implicated in the formation of PML IV bodies in our cell culture model, or whether activation of CRAG by experimental stress can convert endogenous PML bodies that do not degrade ATXN7 to degradation-competent bodies, in the absence of overexpression or ß-INF treatment.

Our study with cadmium showed, for the first time, that some endogenous PML bodies, presumably endogenous clastosomes, actively participate in the degradation of mutant ATXN7 and, when disrupted, increase the amount of ATXN7 in cells. The slight increase in the amount of ATXN7 observed when PML III was overexpressed might also explain the disorganization of endogenous clastosomes or a change in their composition. These observations also suggest that endogenous clastosomes are not sufficiently active to prevent the aggregation of mutant ATXN7. The inhibitory effects on aggregation of increased PML IV expression, raises the question as to whether this might provide the basis of an interesting therapeutic strategy to treat SCA7 or other polyQ disorders.

In the brains of patients, endogenous clastosomes might prevent the accumulation of mutant proteins for several decades prior to onset of aggregation. Later in the pathogenic process, the balance between clastosome activity and the level of accumulating polyQ proteins might be altered, due to the disease process or to a decrease in proteasome or chaperone activity during aging (Grune et al., 2004; Proctor et al., 2005), leading to the formation of polyQ aggregates. Since the mutant proteins are concentrated in the clastosomes, this might even be a site where the aggregation of proteolytic fragments of the proteins begins. The colocalization of PML bodies and small polyQ aggregates in the brain of patients and animal models supports this hypothesis.

ß-INF increased the number and size of PML bodies and decreased the levels of both soluble and aggregated mutant ATXN7. Although the debate concerning the relative contributions of soluble and aggregated polyQ proteins to cellular toxicity has not yet been resolved (Michalik and Van Broeckhoven, 2003), the reduction of either form of the protein could be beneficial to cells. The observation that only mutant but not endogenous ATXN7 is degraded in the presence of ß-INF, which is an approved drug used in the treatment of multiple sclerosis (Javed and Reder, 2005), reinforces the hypothesis that this substance may have therapeutic value, since it would not eliminate functional ATXN7.

### References :

Beech, S.J., K.J. Lethbridge, N. Killick, N. McGlincy, and K.N. Leppard. 2005. Isoforms of the promyelocytic leukemia protein differ in their effects on ND10 organization. Exp Cell Res. 307:109-17.

Bischof, O., O. Kirsh, M. Pearson, K. Itahana, P.G. Pelicci, and A. Dejean. 2002. Deconstructing PML-induced premature senescence. Embo J. 21:3358-69.

Borden, K.L. 2002. Pondering the promyelocytic leukemia protein (PML) puzzle: possible functions for PML nuclear bodies. Mol Cell Biol. 22:5259-69.

Cancel, G., C. Duyckaerts, M. Holmberg, C. Zander, G. Yvert, A.S. Lebre, M. Ruberg, B. Faucheux, Y. Agid, E. Hirsch, and A. Brice. 2000. Distribution of ataxin-7 in normal human brain and retina. Brain. 123 Pt 12:2519-30.

Chai, Y., S.L. Koppenhafer, S.J. Shoesmith, M.K. Perez, and H.L. Paulson. 1999. Evidence for proteasome involvement in polyglutamine disease: localization to nuclear inclusions in SCA3/MJD and suppression of polyglutamine aggregation in vitro. Hum Mol Genet. 8:673-82.

Charlier M, L'Haridon R, Boisnard M, Martal J, Gaye P Cloning and structural analysis of four genes encoding interferon-omega in rabbit. J Interferon Res. 1993 Oct;13(5):313-22.

Chelbi-Alix MK, Pelicano L, Quignon F, Koken MH, Venturini L, Stadler M, Pavlovic J, Degos L, de The H. Induction of the PML protein by interferons in normal and APL cells. Leukemia. 1995 Dec;9(12):2027-33.

Coutts A S, La Thangue N B, BBRC, 2005, 331, 778-785, The p53 response: emerging levels of co-factor complexity.

Daniel MT, Koken M, Romagne O, Barbey S, Bazarbachi A, Stadler M, Guillemin MC, Degos L, Chomienne C, de The H. PML protein expression in hematopoietic and acute promyelocytic leukemia cells. Blood. 1993 Sep 15;82(6):1858-67.

David, G., N. Abbas, G. Stevanin, A. Durr, G. Yvert, G. Cancel, C. Weber, G. Imbert, F. Saudou, E. Antoniou, H. Drabkin, R. Gemmill, P. Giunti, A. Benomar, N. Wood, M. Ruberg, Y. Agid, J.L. Mandel, and A. Brice. 1997. Cloning of the SCA7 gene reveals a highly unstable CAG repeat expansion. Nat Genet. 17:65-70.

de Stanchina E, Querido E, Narita M, .Pandolfi P P, Ferbeyre G and Lowe S W .Molecular Cell ,2004, vol. 13, 523-535 PML is a direct p53 target that modulates p53 effector functions.

de The H, Lavau C, Marchio A, Chomienne C, Degos L, Dejean A The PML-RAR alpha fusion mRNA generated by the t(15;17) translocation in acute promyelocytic leukemia encodes a functionally altered RAR. Cell. 1991 Aug 23;66(4):675-84.

Dyck JA, Maul GG, Miller WH Jr, Chen JD, Kakizuka A, Evans RM. A novel macromolecular structure is a target of the promyelocyte-retinoic acid receptor oncoprotein. Cell. 1994 Jan 28;76(2):333-43.

Everett R D: Microreview , Cellular Microbiology, 2006, vol 8, 365-374. Interactions between DNA viruses, ND10 and the DNA damage response

Fagioli M, Alcalay M, Pandolfi PP, Venturini L, Mencarelli A, Simeone A, Acampora D, Grignani F, Pelicci PG. Alternative splicing of PML transcripts predicts coexpression of several carboxy-terminally different protein isoforms. Oncogene. 1992 Jun;7(6):1083-91.

Fogal, V., M. Gostissa, P. Sandy, P. Zacchi, T. Sternsdorf, K. Jensen, P.P. Pandolfi, H. Will, C. Schneider, and G. Del Sal. 2000. Regulation of p53 activity in nuclear bodies by a specific PML isoform. Embo J. 19:6185-95.

Fujigasaki et al 2001. H. Fujigasaki, J.J. Martin, P.P. De Deyn, A. Camuzat, D. Deffond, G. Stevanin, B. Dermaut, C. Van Broeckhoven, A. Dürr and A. Brice, CAG repeat expansion in the TATA box-binding protein gene causes autosomal dominant TATA box-binding protein gene causes autosomal dominant cerebellar ataxia. Brain 124 (2001), pp. 1939-1947.

Goddard AD, Borrow J, Freemont PS, Solomon E. Characterization of a zinc finger gene disrupted by the t(15;17) in acute promyelocytic leukemia. Science. 1991 Nov 29;254(5036):1371-4.

Goti, D., S.M. Katzen, J. Mez, N. Kurtis, J. Kiluk, L. Ben-Haiem, N.A. Jenkins, N.G. Copeland, A. Kakizuka, A.H. Sharp, C.A. Ross, P.R. Mouton, and V. Colomer. 2004. A mutant ataxin-3 putative-cleavage fragment in brains of Machado-Joseph disease patients and transgenic mice is cytotoxic above a critical concentration. J Neurosci. 24:10266-79.

Grignani, F., U. Testa, D. Rogaia, P.F. Ferrucci, P. Samoggia, A. Pinto, D. Aldinucci, V. Gelmetti, M. Fagioli, M. Alcalay, J. Seeler, I. Nicoletti, C. Peschle, and P.G. Pelicci. 1996. Effects on differentiation by the promyelocytic leukemia PML/RARalpha protein depend on the fusion of the PML protein dimerization and RARalpha DNA binding domains. Embo J. 15:4949-58.

Grune, T., T. Jung, K. Merker, and K.J. Davies. 2004. Decreased proteolysis caused by protein aggregates, inclusion bodies, plaques, lipofuscin, ceroid, and 'aggresomes' during oxidative stress, aging, and disease. Int J Biochem Cell Biol. 36:2519-30.

Hackam AS, Singaraja R, Wellington CL, Metzler M, McCutcheon K, Zhang T, Kalchman M, Hayden MR. The influence of huntingtin protein size on nuclear localization and cellular toxicity. J Cell Biol. 1998 Jun 1;141(5):1097-105.

Helmlinger, D., G. Abou-Sleymane, G. Yvert, S. Rousseau, C. Weber, Y. Trottier, J.L. Mandel, and D. Devys. 2004a. Disease progression despite early loss of polyglutamine protein expression in SCA7 mouse model. J Neurosci. 24:1881-7.

Helmlinger, D., S. Hardy, S. Sasorith, F. Klein, F. Robert, C. Weber, L. Miguet, N. Potier, A. Van-Dorsselaer, J.M. Wurtz, J.L. Mandel, L. Tora, and D. Devys. 2004b. Ataxin-7 is a subunit of GCN5 histone acetyltransferase-containing complexes. Hum Mol Genet. 13:1257-65.

Helmlinger, D., S. Hardy, G. Abou-Sleymane, A. Eberlin, A.B. Bowman, A. Gansmuller, S. Picaud, H.Y. Zoghbi, Y. Trottier, L. Tora, and D. Devys. 2006. Glutamine-Expanded Ataxin-7 Alters TFTC/STAGA Recruitment and Chromatin Structure Leading to Photoreceptor Dysfunction. PLoS Biol. 4:e67.

Isaacs A, Lindenmann J. Virus interference. I. The interferon. Proc R Soc Lond B Biol Sci. 1957 Sep 12;147(927):258-67.

Javed, A., and A.T. Reder. 20065. Therapeutic role of beta-interferons in multiple sclerosis. Pharmacol Ther.

Jensen, K., C. Shiels, and P.S. Freemont. 2001. PML protein isoforms and the RBCC/TRIM motif. Oncogene. 20:7223-33.

Kakizuka A, Miller WH Jr, Umesono K, Warrell RP Jr, Frankel SR, Murty VV, Dmitrovsky E, Evans RM Chromosomal translocation t(15;17) in human acute promyelocytic leukemia fuses RAR alpha with a novel putative transcription factor, PML. Cell. 1991 Aug 23;66(4):663-74.

Kastner, P., A. Perez, Y. Lutz, C. Rochette-Egly, M.P. Gaub, B. Durand, M. Lanotte, R. Berger, and P. Chambon. 1992. Structure, localization and transcriptional properties of two classes of retinoic acid receptor alpha fusion proteins in acute promyelocytic leukemia (APL): structural similarities with a new family of oncoproteins. Embo J. 11:629-42.

Kaytor, M.D., L.A. Duvick, P.J. Skinner, M.D. Koob, L.P. Ranum, and H.T. Orr. 1999. Nuclear localization of the spinocerebellar ataxia type 7 protein, ataxin-7. Hum Mol Genet. 8:1657-64.

Koken MH, Puvion-Dutilleul F, Guillemin MC, Viron A, Linares-Cruz G, Stuurman N, de Jong L, Szostecki C, Calvo F, Chomienne C, The t(15;17) translocation alters a nuclear body in a retinoic acid-reversible fashion. EMBO J. 1994 Mar 1;13(5):1073-83.

Kruse, M.L., A. Arlt, A. Sieke, F. Grohmann, M. Grossmann, J. Minkenberg, U.R. Folsch, and H. Schafer. 2005. Immediate early gene X1 (IEX-1) is organized in subnuclear structures and partially co-localizes with promyelocytic leukemia protein in HeLa cells. J Biol Chem. 280:24849-56.

La Spada, A.R., Y.H. Fu, B.L. Sopher, R.T. Libby, X. Wang, L.Y. Li, D.D. Einum, J. Huang, D.E. Possin, A.C. Smith, R.A. Martinez, K.L. Koszdin, P.M. Treuting, C.B. Ware, J.B. Hurley, L.J. Ptacek, and S. Chen. 2001. Polyglutamine-expanded ataxin-7 antagonizes CRX function and induces cone-rod dystrophy in a mouse model of SCA7. Neuron. 31:913-27.

Lafarga, M., M.T. Berciano, E. Pena, I. Mayo, J.G. Castano, D. Bohmann, J.P. Rodrigues, J.P. Tavanez, and M. Carmo-Fonseca. 2002. Clastosome: a subtype of nuclear body enriched in 19S and 20S proteasomes, ubiquitin, and protein substrates of proteasome. Mol Biol Cell. 13:2771-82.

Lavau C, Marchio A, Fagioli M, Jansen J, Falini B, Lebon P, Grosveld F, Pandolfi PP, Pelicci PG, Dejean A. The acute promyelocytic leukaemia-associated PML gene is induced by interferon. Oncogene. 1995 Sep 7;11(5):871-6.Lavau et al 1995, Oncogene, 11,871-876)

Lin LS, Yamamoto R, Drummond RJ. Purification of recombinant human interferon beta expressed in Escherichia coli. Methods Enzymol. 1986;119:183-92.

McMahon, S.J., M.G. Pray-Grant, D. Schieltz, J.R. Yates, 3rd, and P.A. Grant. 2005. Polyglutamine-expanded spinocerebellar ataxia-7 protein disrupts normal SAGA and SLIK histone acetyltransferase activity. Proc Natl Acad Sci U S A. 102:8478-82.

Michalik, A., and C. Van Broeckhoven. 2003. Pathogenesis of polyglutamine disorders: aggregation revisited. Hum Mol Genet. 12 Spec No 2:R173-86.

Môller A, Sirma H, Hofmann T G, Rueffer S,Klimczak E, Drôge W, Will H,and Schmitz M L. PML is required for Homeodomain-interacting Protein kinase 2 (HIPK2)-mediated p53 phosphorylation and cell cycle arrest but is dispensable for the formation of HIPK domains. Cancer research, 2003, vol 63, 4310-4314.

Muratani, M., D. Gerlich, S.M. Janicki, M. Gebhard, R. Eils, and D.L. Spector. 2002. Metabolic-energy-dependent movement of PML bodies within the mammalian cell nucleus. Nat Cell Biol. 4:106-10.

Nakamura K, Jeong SY, Uchihara T, Anno M, Nagashima K, Nagashima T, Ikeda S, Tsuji S, Kanazawa I. SCA17, a novel autosomal dominant cerebellar ataxia caused by an expanded polyglutamine in TATA-binding protein. Hum Mol Genet. 2001 Jul 1;10(14):1441-8.

Nefkens, I., D.G. Negorev, A.M. Ishov, J.S. Michaelson, E.T. Yeh, R.M. Tanguay, W.E. Muller, and G.G. Maul. 2003. Heat shock and Cd2+ exposure regulate PML and Daxx release from ND10 by independent mechanisms that modify the induction of heat-shock proteins 70 and 25 differently. J Cell Sci. 116:513-24.

Negorev, D., and G.G. Maul. 2001. Cellular proteins localized at and interacting within ND10/PML nuclear bodies/PODs suggest functions of a nuclear depot. Oncogene. 20:7234-42.

Palhan, V.B., S. Chen, G.H. Peng, A. Tjernberg, A.M. Gamper, Y. Fan, B.T. Chait, A.R. La Spada, and R.G. Roeder. 2005. Polyglutamine-expanded ataxin-7 inhibits STAGA histone acetyltransferase activity to produce retinal degeneration. Proc Natl Acad Sci U S A. 102:8472-7.

Perez MK, Paulson HL, Pendse SJ, Saionz SJ, Bonini NM, Pittman RN. Recruitment and the role of nuclear localization in polyglutamine-mediated aggregation. J Cell Biol. 1998 Dec 14;143(6):1457-70.

Proctor, C.J., C. Soti, R.J. Boys, C.S. Gillespie, D.P. Shanley, D.J. Wilkinson, and T.B. Kirkwood. 2005. Modelling the actions of chaperones and their role in ageing. Mech Ageing Dev. 126:119-31.

Qin, Q., R. Inatome, A. Hotta, M. Kojima, H. Yamamura, H. Hirai, T. Yoshizawa, H. Tanaka, K. Fukami, and S. Yanagi. 2006. A novel GTPase, CRAG, mediates promyelocytic leukemia protein-associated nuclear body formation and degradation of expanded polyglutamine protein. J Cell Biol. 172:497-504.

Regad, T., and M.K. Chelbi-Alix. 2001. Role and fate of PML nuclear bodies in response to interferon and viral infections. Oncogene. 20:7274-86.

Reymond A, Meroni G, Fantozzi A, Merla G, Cairo S, Luzi L, Riganelli D, Zanaria E, Messali S, Cainarca S, Guffanti A, Minucci S, Pelicci PG, Ballabio A. The tripartite motif family identifies cell compartments. EMBO J. 2001 May 1;20(9):2140-51.

Rockel, T.D., D. Stuhlmann, and A. von Mikecz. 2005. Proteasomes degrade proteins in focal subdomains of the human cell nucleus. J Cell Sci. 118:5231-42.

Rueda BR, Naivar KA, George EM, Austin KJ, Francis H, Hansen TR. Recombinant interferon-tau regulates secretion of two bovine endometrial proteins. J Interferon Res. 1993 Aug;13(4):303-9.

Sanchez, I., C. Mahlke, and J. Yuan. 2003. Pivotal role of oligomerization in expanded polyglutamine neurodegenerative disorders. Nature. 421:373-9.

Scheel, H., S. Tomiuk, and K. Hofmann. 2003. Elucidation of ataxin-3 and ataxin-7 function by integrative bioinformatics. Hum Mol Genet. 12:2845-52.

Scherzinger E, Lurz R, Turmaine M, Mangiarini L, Hollenbach B, Hasenbank R, Bates GP, Davies SW, Lehrach H, Wanker EE. Huntingtin-encoded polyglutamine expansions form amyloid-like protein aggregates in vitro and in vivo. Cell. 1997 Aug 8;90(3):549-58.

Seeler, J.S., and A. Dejean. 1999. The PML nuclear bodies: actors or extras? Curr Opin Genet Dev. 9:362-7.

Sittler, A., D. Devys, C. Weber, and J.L. Mandel. 1996. Alternative splicing of exon 14 determines nuclear or cytoplasmic localisation of fmr1 protein isoforms. Hum Mol Genet. 5:95-102.

Sittler, A., S. Walter, N. Wedemeyer, R. Hasenbank, E. Scherzinger, H. Eickhoff, G.P. Bates, H. Lehrach, and E.E. Wanker. 1998. SH3GL3 associates with the Huntingtin exon 1 protein and promotes the formation of polygln-containing protein aggregates. Mol Cell. 2:427-36.

Skinner, P.J., B.T. Koshy, C.J. Cummings, I.A. Klement, K. Helin, A. Servadio, H.Y. Zoghbi, and H.T. Orr. 1997. Ataxin-1 with an expanded glutamine tract alters nuclear matrix- associated structures. Nature. 389:971-4.

Stevanin, G., A. Durr, and A. Brice. 2002. Spinocerebellar ataxias caused by polyglutamine expansions. Adv Exp Med Biol. 516:47-77.

Takahashi, J., H. Fujigasaki, C. Zander, K.H. El Hachimi, G. Stevanin, A. Durr, A.S. Lebre, G. Yvert, Y. Trottier, H. The, J.J. Hauw, C. Duyckaerts, and A. Brice. 2002. Two populations of neuronal intranuclear inclusions in SCA7 differ in size and promyelocytic leukaemia protein content. Brain. 125:1534-43.

Takahashi, J., H. Fujigasaki, K. Iwabuchi, A.C. Bruni, T. Uchihara, K.H. El Hachimi, G. Stevanin, A. Durr, A.S. Lebre, Y. Trottier, H. de The, J. Tanaka, J.J. Hauw, C. Duyckaerts, and A. Brice. 2003. PML nuclear bodies and neuronal intranuclear inclusion in polyglutamine diseases. Neurobiol Dis. 13:230-7.

Tanaka, M., Y. Machida, S. Niu, T. Ikeda, N.R. Jana, H. Doi, M. Kurosawa, M. Nekooki, and N. Nukina. 2004. Trehalose alleviates polyglutamine-mediated pathology in a mouse model of Huntington disease. Nat Med. 10:148-54.

The Huntington's Disease Collaborative Research Group. Cell. A novel gene containing a trinucleotide repeat that is expanded and unstable on Huntington's disease chromosomes. The Huntington's Disease Collaborative Research Group. Cell. 1993 Mar 26;72(6):971-83.

Wanker, E.E., E. Scherzinger, V. Heiser, A. Sittler, H. Eickhoff, and H. Lehrach. 1999. Membrane filter assay for detection of amyloid-like polyglutamine-containing protein aggregates. Methods Enzymol. 309:375-86.

Watase, K., E.J. Weeber, B. Xu, B. Antalffy, L. Yuva-Paylor, K. Hashimoto, M. Kano, R. Atkinson, Y. Sun, D.L. Armstrong, J.D. Sweatt, H.T. Orr, R. Paylor, and H.Y. Zoghbi. 2002. A long CAG repeat in the mouse Sca1 locus replicates SCA1 features and reveals the impact of protein solubility on selective neurodegeneration. Neuron. 34:905-19.

Waza, M., H. Adachi, M. Katsuno, M. Minamiyama, C. Sang, F. Tanaka, A. Inukai, M. Doyu, and G. Sobue. 2005. 17-AAG, an Hsp90 inhibitor, ameliorates polyglutamine-mediated motor neuron degeneration. Nat Med. 11:1088-95.

Yoo, S.Y., M.E. Pennesi, E.J. Weeber, B. Xu, R. Atkinson, S. Chen, D.L. Armstrong, S.M. Wu, J.D. Sweatt, and H.Y. Zoghbi. 2003. SCA7 knockin mice model human SCA7 and reveal gradual accumulation of mutant ataxin-7 in neurons and abnormalities in short-term plasticity. Neuron. 37:383-401.

Yvert, G., K.S. Lindenberg, D. Devys, D. Helmlinger, G.B. Landwehrmeyer, and J.L. Mandel. 2001. SCA7 mouse models show selective stabilization of mutant ataxin-7 and similar cellular responses in different neuronal cell types. Hum Mol Genet. 10:1679-92.

Yvert, G., K.S. Lindenberg, S. Picaud, G.B. Landwehrmeyer, J.A. Sahel, and J.L. Mandel. 2000. Expanded polyglutamines induce neurodegeneration and transneuronal alterations in cerebellum and retina of SCA7 transgenic mice. Hum Mol Genet. 9:2491-506.

Zander C, Takahashi J, El Hachimi KH, Fujigasaki H, Albanese V, Lebre AS, Stevanin G, Duyckaerts C, Brice A. 2001. Similarities between spinocerebellar ataxia type 7 (SCA7) cell models and human brain: proteins recruited in inclusions and activation of caspase-3. Hum Mol Genet. 10: 2569-2579.

Zhong, S., P. Salomoni, and P.P. Pandolfi. 2000. The transcriptional role of PML and the nuclear body. Nat Cell Biol. 2:E85-90.

Zoghbi and Orr 2000. H.Y. Zoghbi and H.T. Orr, Glutamine repeats and neurodegeneration. Annu. Rev. Neurosci. 23 (2000), pp. 217-247.

## Claims

1. Use of an inducer of PML (promyelocytic leukemia protein) expression for the manufacture of a medicament intended for treating a polyglutamine expansion neurodegenerative disease.

2. The use of claim 1, wherein the inducer is an inducer of PML IV.

3. The use according to claim 1 or 2 wherein said inducer is an interferon.

4. The use of claim 3, wherein the interferon is interferon beta.

5. The use of claim 4, wherein the interferon is interferon beta 1 a.

6. The use of claim 3, wherein the interferon is interferon alpha.

7. Use according to any of claims 1 to 6 wherein a polyglutamine expansion neurodegenerative disease is selected from the group consisting of spinocerebellar ataxia 1, 2, 3, 6, 7, and 17, Huntington's disease, spinal and bulbar muscular atrophy, and dentatorubral pallidoluysian atrophy.
